# EUROPÄISCHE PATENTSCHRIFT

(11) **EP 3 230 428 B1**
(45) Veröffentlichungstag und Bekanntmachung des Hinweises auf die Patenterteilung: **26.09.2018**
(21) Anmeldenummer: 15804750.6
(22) Anmeldetag: 02.12.2015
(51) Int. Cl.: C11D 3/386

(54) **HANDGESCHIRRSPÜLMITTEL MIT VERBESSERTER WIRKUNG GEGEN STÄRKE**
HAND DISHWASHING DETERGENT HAVING AN IMPROVED EFFECT AGAINST STARCH
DÉTERGENT POUR LAVAGE MANUEL DE MANUEL, À ACTION AMÉLIORÉE CONTRE AMIDON

(30) Priorität: 10.12.2014 DE 102014225472
(43) Veröffentlichungstag der Anmeldung: 18.10.2017
(73) Patentinhaber: Henkel AG & Co. KGaA, 40589 Düsseldorf (DE)
(72) Erfinder: PEGELOW, Ulrich, 40597 Düsseldorf (DE); BUISKER, Detlef, 45134 Essen (DE); O'CONNELL, Timothy, 86899 Landsberg am Lech (DE); RASCHKE, Ines, 40764 Langenfeld (DE)
(86) Internationale Anmeldenummer: PCT/EP2015/078400
(87) Internationale Veröffentlichungsnummer: WO 2016/091688

(56) Entgegenhaltungen:
- WO-A1-2014/183920
- CA-A- 908 079
- US-A1- 2011 240 510

### SEQUENCE LISTING

<110> Henkel AG & Co. KGaA
<120> Handgeschirrspülmittel mit verbesserter Wirkung gegen Stärke
<130> PT032570_PCT
<150> 102014225472.8
   <151> 2014-12-10
<160> 1
<170> PatentIn version 3.5
<210> 1
   <211> 485
   <212> PRT
   <213> Artificial
<220>
   <223> Hybridprotein
<400> 1
   His His Asn Gly Thr Asn Gly Thr Met Met Gln Tyr Phe Glu Trp Tyr
   1               5                   10                  15
   Leu Pro Asn Asp Gly Asn His Trp Asn Arg Leu Asn Ser Asp Ala Ser
               20                  25                  30
   Asn Leu Lys Ser Lys Gly Ile Thr Ala Val Trp Ile Pro Pro Ala Trp
           35                  40                  45
   Lys Gly Ala Ser Gln Asn Asp Val Gly Tyr Gly Ala Tyr Asp Leu Tyr
       50                  55                  60
   Asp Leu Gly Glu Phe Asn Gln Lys Gly Thr Val Arg Thr Lys Tyr Gly
   65                  70                  75                  80
   Thr Arg Ser Gln Leu Gln Ala Ala Val Thr Ser Leu Lys Asn Asn Gly
                   85                  90                  95
   Ile Gln Val Tyr Gly Asp Val Val Met Asn His Lys Gly Gly Ala Asp
               100                 105                 110
   Ala Thr Glu Met Val Arg Ala Val Glu Val Asn Pro Asn Asn Arg Asn
           115                 120                 125
   Gln Glu Val Thr Gly Glu Tyr Thr Ile Glu Ala Trp Thr Arg Phe Asp
       130                 135                 140
   Phe Pro Gly Arg Gly Asn Thr His Ser Ser Phe Lys Trp Arg Trp Tyr
   145                 150                 155                 160
   His Phe Asp Gly Val Asp Trp Asp Gln Ser Arg Arg Leu Asn Asn Arg
                   165                 170                 175
   Ile Tyr Lys Phe Arg Gly His Gly Lys Ala Trp Asp Trp Glu Val Asp
               180                 185                 190
   Thr Glu Asn Gly Asn Tyr Asp Tyr Leu Met Tyr Ala Asp Ile Asp Met
           195                 200                 205
   Asp His Pro Glu Val Val Asn Glu Leu Arg Asn Trp Gly Val Trp Tyr
       210                 215                 220
   Thr Asn Thr Leu Gly Leu Asp Gly Phe Arg Ile Asp Ala Val Lys His
   225                 230                 235                 240
   Ile Lys Tyr Ser Phe Thr Arg Asp Trp Ile Asn His Val Arg Ser Ala
                   245                 250                 255
   Thr Gly Lys Asn Met Phe Ala Val Ala Glu Phe Trp Lys Asn Asp Leu
               260                 265                 270
   Gly Ala Ile Glu Asn Tyr Leu Gln Lys Thr Asn Trp Asn His Ser Val
           275                 280                 285
   Phe Asp Val Pro Leu His Tyr Asn Leu Tyr Asn Ala Ser Lys Ser Gly
       290                 295                 300
   Gly Asn Tyr Asp Met Arg Asn Ile Phe Asn Gly Thr Val Val Gln Arg
   305                 310                 315                 320
   His Pro Ser His Ala Val Thr Phe Val Asp Asn His Asp Ser Gln Pro
                   325                 330                 335
   Glu Glu Ala Leu Glu Ser Phe Val Glu Glu Trp Phe Lys Pro Leu Ala
               340                 345                 350
   Tyr Ala Leu Thr Leu Thr Arg Glu Gln Gly Tyr Pro Ser Val Phe Tyr
           355                 360                 365
   Gly Asp Tyr Tyr Gly Ile Pro Thr His Gly Val Pro Ala Met Arg Ser
       370                 375                 380
   Lys Ile Asp Pro Ile Leu Glu Ala Arg Gln Lys Tyr Ala Tyr Gly Pro
   385                 390                 395                 400
   Gln His Asp Tyr Ile Asp His Pro Asp Val Ile Gly Trp Thr Arg Glu
                       405                 410                 415
   Gly Asp Ser Ser Ala Ala Lys Ser Gly Leu Ala Ala Leu Ile Thr Asp
                   420                 425                 430
   Gly Pro Gly Gly Ser Lys Arg Met Tyr Ala Gly Leu Lys Asn Ala Gly
               435                 440                 445
   Glu Thr Trp Tyr Asp Ile Thr Gly Asn Arg Ser Asp Thr Val Lys Ile
       450                 455                 460
   Gly Ser Asp Gly Trp Gly Glu Phe His Val Asn Asp Gly Ser Val Ser
   465                 470                 475                 480
   Ile Tyr Val Gln Lys
                   485

## Beschreibung

Die vorliegende Erfindung betrifft ein flüssiges Reinigungsmittel, insbesondere ein wässriges Reinigungsmittel für harte Oberflächen. Das Reinigungsmittel wird bevorzugt zum manuellen Geschirrspülen eingesetzt.

Übliche flüssige Reinigungsmittel enthalten Tenside zur Entfernung von Schmutz und Flecken. In der Regel werden hierbei Kombinationen aus mehreren Tensiden, insbesondere aus der Gruppe der anionischen, nichtionischen, kationischen und amphoteren Tenside verwendet. Diese Tenside allein sind häufig nicht in der Lage, Schmutz und Flecken hinreichend zu entfernen, so dass in modernen Wasch- oder Reinigungsmitteln weitere Hilfsstoffe eingesetzt werden. Zu diesen weiteren Hilfsstoffen gehören Enzyme verschiedener Arten wie Proteasen, Amylasen, Cellulasen, Mannanasen oder Pektatlyasen. Dem Fachmann sind weitere Enzymklassen bekannt. Dabei kommt den Amylasen aufgrund ihrer Wirksamkeit gegen eingetrocknete Stärke eine besondere Bedeutung zu, da durch ihren Einsatz eine erleichterte Reinigung bei geringerem mechanischem Aufwand erzielt wird.

WO2014/183920 A1 offenbart ein flüssiges Reinigungsmittel für harte Oberflächen, insbesondere zum manuellen Geschirrspülen, enthaltend eine modifizierte α-Amylase mit Deletionen H183* und G184*.

Die Leistung der bislang in Handgeschirrspülmitteln eingesetzten Amylasen ist jedoch stark temperaturabhängig, so dass die Vorteile dieser Wirkstoffe, die sich bei Spültemperaturen um 40°C entfalten, bei geringeren Temperaturen erheblich abgeschwächt werden und bei 20°C kaum noch eine Wirkung erkennbar ist. Andererseits ist es jedoch aus Gründen der Nachhaltigkeit und der Energieeffizienz wünschenswert, dass auch bei niedrigen Temperaturen eine wirksame Einweichleistung gegenüber Stärke vorliegt.

Der Erfindung liegt daher die Aufgabe zugrunde, ein flüssiges Reinigungsmittel, insbesondere für harte Oberflächen, bereitzustellen, das auch bei niedrigen Temperaturen eine gute Reinigungsleistung gegenüber stärkehaltigen, auch eingetrockneten Anschmutzungen aufweist. Überraschenderweise wurde gefunden, dass diese Aufgabe gelöst wird durch ein flüssiges Reinigungsmittel enthaltend eine α-Amylase, die zu der in SEQ ID NO.1 angegeben Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 Deletionen aufweist. Mit einem solchen Reinigungsmittel lassen sich auch bei Temperaturen deutlich unterhalb von 40°C bereits sehr gute Reinigungsergebnisse insbesondere bei der Stärkeentfernung erzielen.

Gegenstand der vorliegenden Erfindung ist daher ein flüssiges Reinigungsmittel enthaltend eine α-Amylase, die zu der in SEQ ID NO.1 angegeben Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 Deletionen aufweist, wobei die α-Amylase Deletionen an mindestens zwei Positionen ausgewählt aus den Positionen 180+181, 181+182, 182+183 und 183+184 in der Zählung gemäß SEQ ID NO. 1 aufweist, und wobei das flüssige Reinigungsmittel Kaliumionen enthält und der Anteil der Kaliumionen an der Gesamtzusammensetzung 0,025 bis 0,25 Gew.-% beträgt. Ganz besonders bevorzugt sind Deletionen an den Positionen 183+184 in der Zählung gemäß SEQ ID NO. 1, insbesondere bevorzugt die Deletionen H183* + G184*. Vorzugsweise weist die erfindungsgemäße α-Amylase in der Zählung gemäß SEQ ID NO. 1 weiterhin eine Aminosäuresubstitution an einer oder mehreren der Positionen 405, 421, 422 und 428 auf. Besonders bevorzugt sind die Substitutionen I405L; A421H, A422P und A428T.

In einer besonders bevorzugten Ausführungsform weist die erfindungsgemäße α-Amylase in der Zählung gemäß SEQ ID NO. 1 die Deletionen H183* + G184* und zusätzlich die Substitutionen I405L, A421H, A422P und A428T auf.

Aminosäurepositionen, die im Rahmen der vorliegenden Erfindung mit der Formulierung "Zählung gemäß SEQ ID NO. 1" angegeben werden, verstehen sich folgendermaßen: Die weiteren Aminosäurepositionen werden durch ein Alignment der Aminosäuresequenz einer erfindungsgemäßen Amylase mit der Aminosäuresequenz, wie sie in SEQ ID NO. 1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz eines erfindungsgemäßen Proteins eine höhere Zahl von Aminosäureresten umfasst als die Amylase in SEQ ID NO. 1. Ausgehend von den genannten Positionen in der Aminosäuresequenz sind die Veränderungspositionen in einer erfindungsgemäßen Amylase diejenigen, die eben diesen Positionen in einem Alignement zugeordnet sind.

Ein weiterer Gegenstand der vorliegenden Erfindung ist die Verwendung eines erfindungsgemäßen flüssigen Reinigungsmittels zum manuellen Spülen von Geschirr. Noch ein weiterer Gegenstand der vorliegenden Erfindung ist ein Verfahren zur Reinigung von harten Oberflächen, insbesondere zur manuellen Reinigung von Geschirr, umfassend die Verwendung eines erfindungsgemäßen flüssigen Reinigungsmittels.

Im Rahmen der vorliegenden Erfindung besonders bevorzugt sind flüssige Reinigungsmittel mit einem Gehalt an α-Amylase von 0,05 bis 1,0 Gew.-%, vorzugsweise von 0,1 bis 0,4 Gew.-%. Unter flüssigen Reinigungsmitteln werden im Rahmen der vorliegenden Erfindung solche verstanden, die unter normalen Anwendungsbedingungen fließfähig sind und deren Viskositäten in einem breiten Rahmen variieren können. Zu den flüssigen Zubereitungen zählen auch gelförmige oder pastöse Mittel, welche gegebenenfalls zusätzliche aus dem Stand der Technik bekannte Verdickungsmittel aufweisen können. In einer weiter bevorzugten Ausführungsform der Erfindung beruhen die flüssigen Mittel auf wässeriger Basis. Insbesondere handelt es sich bei den erfindungsgemäßen Reinigungsmitteln um Handgeschirrspülmittel.

Ein Handgeschirrspülmittel (synonym: manuelles Geschirrspülmittel) ist im Kontext der vorliegenden Erfindung ein flüssiges Wasch- oder Reinigungsmittel, das an die Verwendung zum manuellen Spülen von Geschirr besonders angepasst ist. Handgeschirrspülmittel sind demnach besonders dazu geeignet, Schmutz von harten Oberflächen zu lösen, weisen ein gutes Schaumvermögen und zudem eine besondere Hautverträglichkeit auf.

Vorteilhafterweise ist das Reinigungsmittel nach der vorliegenden Erfindung bevorzugt als Handgeschirrspülmittel ausgebildet, das ein Schaumvermögen von mindestens 250 mL, gemessen nach der DIN-Methode 53 902, Teil 2 (Ross-Miles-Test), vorzugsweise von mindestens 300 mL aufweist. Dieses vorteilhafte Schaumverhalten ist typischerweise darauf zurückzuführen, dass das Handgeschirrspülmittel vorzugsweise mindestens 5% Gew.-% eines anionischen Tensids, bezogen auf das gesamte Handgeschirrspülmittel, enthält. Der Gesamtgehalt an Tensid kann allerdings deutlich darüber liegen (siehe unten).

Um einerseits eine gute Hautverträglichkeit zu gewährleisten und andererseits eine optimale Wirksamkeit der enthaltenen Enzyme zu ermöglichen, liegt der pH Wert des Handgeschirrspülmittels vorzugsweise im Bereich von (jeweils einschließlich) 6,0 bis 9,0. Besonders bevorzugt sind pH Werte zwischen pH 7,0 und pH 8,0 (jeweils einschließlich).

Die erfindungsgemäßen Reinigungsmittel enthalten als weitere Bestandteile üblicherweise Tenside, vor allem anionische Tenside, nichtionische Tenside, amphotere Tenside, Betaine sowie gegebenenfalls kationische Tenside. Die Gesamtmenge der Tenside in den erfindungsgemäßen Mitteln kann in einem breiten Rahmen variieren und beispielsweise 3 bis 70 Gew.-%, vorzugsweise 5 bis 50 Gew.-% und insbesondere 10 bis 40 Gew.-% betragen.
Die anionischen Tenside werden üblicherweise als Alkalimetall-, Erdalkalimetall- und/oder Mono-, Di- bzw. Trialkanolammoniumsalz und/oder aber auch in Form ihrer mit dem entsprechenden Alkalimetallhydroxid, Erdalkalimetallhydroxid und/oder Mono-, Di- bzw. Trialkanolamin in situ zu neutralisierenden korrespondierenden Säure eingesetzt. Bevorzugt sind hierbei als Alkalimetalle Kalium und insbesondere Natrium, als Erdalkalimetalle Calcium und insbesondere Magnesium, sowie als Alkanolamine Mono-, Di- oder Triethanolamin. Besonders bevorzugt sind die Natriumsalze.

Zu den insbesondere in manuellen Geschirrspülmitteln bevorzugt eingesetzten Aniontensiden zählen vor allem Alkylethersulfate und Alkylsulfonate.

Alkylethersulfate (Fettalkoholethersulfate, INCI Alkyl Ether Sulfates) sind Produkte von Sulfatierreaktionen an alkoxylierten Alkoholen. Dabei versteht der Fachmann allgemein unter alkoxylierten Alkoholen die Reaktionsprodukte von Alkylenoxid, bevorzugt Ethylenoxid, mit Alkoholen, im Sinne der vorliegenden Erfindung bevorzugt mit längerkettigen Alkoholen, d.h. mit aliphatischen geradkettigen oder ein oder mehrfach verzweigten, acyclischen oder cyclischen, gesättigten oder ein oder mehrfach ungesättigten, vorzugsweise geradkettigen, acyclischen, gesättigten, Alkoholen mit 6 bis 22, vorzugsweise 8 bis 18, insbesondere 10 bis 16 und besonders bevorzugt 12 bis 14 Kohlenstoffatomen. In der Regel entsteht aus n Molen Ethylenoxid und einem Mol Alkohol, abhängig von den Reaktionsbedingungen, ein komplexes Gemisch von Additionsprodukten unterschiedlicher Ethoxylierungsgrade (n = 1 bis 30, vorzugsweise 1 bis 20, insbesondere 1 bis 10, besonders bevorzugt 2 bis 4). Eine weitere Ausführungsform der Alkoxylierung besteht im Einsatz von Gemischen der Alkylenoxide, bevorzugt des Gemisches von Ethylenoxid und Propylenoxid. Ganz besonders bevorzugt im Sinne der vorliegenden Erfindung sind niederethoxylierte Fettalkohole mit 1 bis 4 Ethylenoxideinheiten (EO), insbesondere 1 bis 2 EO, beispielsweise 2 EO, wie Na-C₁₂₋₁₄-Fettalkohol+2EO-sulfat. In einer bevorzugten Ausführungsform enthält das erfindungsgemäße Mittel mindestens ein Alkylethersulfat.

Die Alkylsulfonate (INCI Sulfonic Acids) weisen üblicherweise einen aliphatischen geradkettigen oder ein- oder mehrfach verzweigten, acyclischen oder cyclischen, gesättigten oder ein- oder mehrfach ungesättigten, vorzugsweise verzweigten, acyclischen, gesättigten, Alkylrest mit 6 bis 22, vorzugsweise 9 bis 20, insbesondere 11 bis 18 und besonders bevorzugt 14 bis 17 Kohlenstoffatomen auf.

Geeignete Alkylsulfonate sind dementsprechend die gesättigten Alkansulfonate, die ungesättigten Olefinsulfonate und die - sich formal von den auch den Alkylethersulfaten zugrunde liegenden alkoxylierten Alkoholen ableitenden - Ethersulfonate, bei denen man endständige Ethersulfonate (n-Ethersulfonate) mit an die Polyether-Kette gebundener Sulfonat-Funktion und innenständige Ethersulfonate (i-Ethersulfonate) mit mit dem Alkylrest verknüpfter Sulfonat-Funktion unterscheidet.

Erfindungsgemäß bevorzugt sind die Alkansulfonate, insbesondere Alkansulfonate mit einem verzweigten, vorzugsweise sekundären, Alkylrest, beispielsweise das sekundäre Alkansulfonat sek. Na-C₁₃₋₁₇-Alkansulfonat (INCI Sodium C14-17 Alkyl Sec Sulfonate).

Weitere mögliche einsetzbare Aniontenside sind dem Fachmann aus dem einschlägigen Stand der Technik zu Wasch- oder Reinigungsmitteln bekannt. Hierzu zählen insbesondere aliphatische Sulfate wie Fettalkoholsulfate, Monoglyceridsulfate sowie Estersulfonate (Sulfofettsäureester), Ligninsulfonate, Alkylbenzolsulfonate, Fettsäurecyanamide, anionische Sulfobernsteinsäuretenside, Fettsäureisethionate, Acylaminoalkansulfonate (Fettsäuretauride), Fettsäuresarcosinate, Ethercarbonsäuren und Alkyl(ether)phosphate.

Geeignete weitere anionische Tenside sind auch anionische Gemini-Tenside mit einer Diphenyloxid-Grundstruktur, 2 Sulfonatgruppen und einem Alkylrest an einem oder beiden Benzolringen gemäß der Formel ⁻O₃S(C₆H₃R)O(C₆H₃R')SO₃⁻, in der R für einen Alkylrest mit beispielsweise 6, 10, 12 oder 16 Kohlenstoffatomen und R' für R oder H steht (Dowfax® Dry Hydrotrope Powder mit C₁₆-Alkylrest(en); INCI Sodium Hexyldiphenyl Ether Sulfonate, Disodium Decyl Phenyl Ether Disulfonate, Disodium Lauryl Phenyl Ether Disulfonate, Disodium Cetyl Phenyl Ether Disulfonate).

Besonders bevorzugte weitere anionische Tenside sind die anionischen Sulfobernsteinsäuretenside Sulfosuccinate, Sulfosuccinamate und Sulfosuccinamide, insbesondere Sulfosuccinate und Sulfosuccinamate, äußerst bevorzugt Sulfosuccinate. Bei den Sulfosuccinaten handelt es sich um die Salze der Mono- und Diester der Sulfobernsteinsäure HOOCCH(SO₃H)CH₂COOH, während man unter den Sulfosuccinamaten die Salze der Monoamide der Sulfobernsteinsäure und unter den Sulfosuccinamiden die Salze der Diamide der Sulfobernsteinsäure versteht. Bei den Salzen handelt es sich bevorzugt um Alkalimetallsalze, Ammoniumsalze sowie Mono-, Di- bzw. Trialkanolammoniumsalze, beispielsweise Mono-, Di- bzw. Triethanolammoniumsalze, insbesondere um Lithium-, Natrium-, Kalium- oder Ammoniumsalze, besonders bevorzugt Natrium- oder Ammoniumsalze, äußerst bevorzugt Natriumsalze.

In den Sulfosuccinaten ist eine bzw. sind beide Carboxylgruppen der Sulfobernsteinsäure vorzugsweise mit einem bzw. zwei gleichen oder verschiedenen unverzweigten oder verzweigten, gesättigten oder ungesättigten, acyclischen oder cyclischen, optional alkoxylierten Alkoholen mit 4 bis 22, vorzugsweise 6 bis 20, insbesondere 8 bis 18, besonders bevorzugt 10 bis 16, äußerst bevorzugt 12 bis 14 Kohlenstoffatomen verestert. Besonders bevorzugt sind die Ester unverzweigter und/oder gesättigter und/oder acyclischer und/oder alkoxylierter Alkohole, insbesondere unverzweigter, gesättigter Fettalkohole und/oder unverzweigter, gesättigter, mit Ethylen- und/oder Propylenoxid, vorzugsweise Ethylenoxid, alkoxylierter Fettalkohole mit einem Alkoxylierungsgrad von 1 bis 20, vorzugsweise 1 bis 15, insbesondere 1 bis 10, besonders bevorzugt 1 bis 6, äußerst bevorzugt 1 bis 4. Die Monoester werden im Rahmen der vorliegenden Erfindung gegenüber den Diestern bevorzugt. Ein besonders bevorzugtes Sulfosuccinat ist Sulfobernsteinsäurelaurylpolyglykolester-di-Natrium-Salz (Lauryl-EO-sulfosuccinat, Di-Na-Salz; INCI Disodium Laureth Sulfosuccinate), das beispielsweise als Tego® Sulfosuccinat F 30 (Goldschmidt) mit einem Sulfosuccinatgehalt von 30 Gew.-% kommerziell erhältlich ist.

In den Sulfosuccinamaten bzw. Sulfosuccinamiden bildet eine bzw. bilden beide Carboxylgruppen der Sulfobernsteinsäure vorzugsweise mit einem primären oder sekundären Amin, das einen oder zwei gleiche oder verschiedene, unverzweigte oder verzweigte, gesättigte oder ungesättigte, acyclische oder cyclische, optional alkoxylierte Alkylreste mit 4 bis 22, vorzugsweise 6 bis 20, insbesondere 8 bis 18, besonders bevorzugt 10 bis 16, äußerst bevorzugt 12 bis 14 Kohlenstoffatomen trägt, ein Carbonsäureamid. Besonders bevorzugt sind unverzweigte und/oder gesättigte und/oder acyclische Alkylreste, insbesondere unverzweigte, gesättigte Fettalkylreste.

Weiterhin geeignet sind beispielsweise die folgenden gemäß INCI bezeichneten Sulfosuccinate und Sulfosuccinamate, die im International Cosmetic Ingredient Dictionary and Handbook näher beschrieben sind: Ammonium Dinonyl Sulfosuccinate, Ammonium Lauryl Sulfosuccinate, Diammonium Dimethicone Copolyol Sulfosuccinate, Diammonium Lauramido-MEA Sulfosuccinate, Diammonium Lauryl Sulfosuccinate, Diammonium Oleamido PEG-2 Sulfosuccinate, Diamyl Sodium Sulfosuccinate, Dicapryl Sodium Sulfosuccinate, Dicyclohexyl Sodium Sulfosuccinate, Diheptyl Sodium Sulfosuccinate, Dihexyl Sodium Sulfosuccinate, Diisobutyl Sodium Sulfosuccinate, Dioctyl Sodium Sulfosuccinate, Disodium Cetearyl Sulfosuccinate, Disodium Cocamido MEA-Sulfosuccinate, Disodium Cocamido MIPA-Sulfosuccinate, Disodium Cocamido PEG-3 Sulfosuccinate, Disodium Coco-Glucoside Sulfosuccinate, Disodium Cocoyl Butyl Gluceth-10 Sulfosuccinate, Disodium C12-15 Pareth Sulfosuccinate, Disodium Deceth-5 Sulfosuccinate, Disodium Deceth-6 Sulfosuccinate, Disodium Dihydroxyethyl Sulfosuccinylundecylenate, Disodium Dimethicone Copolyol Sulfosuccinate, Disodium Hydrogenated Cottonseed Glyceride Sulfosuccinate, Disodium Isodecyl Sulfosuccinate, Disodium Isostearamido MEA-Sulfosuccinate, Disodium Isostearamido MIPA-Sulfosuccinate, Disodium Isostearyl Sulfosuccinate, Disodium Laneth-5 Sulfosuccinate, Disodium Lauramido MEA-Sulfosuccinate, Disodium Lauramido PEG-2 Sulfosuccinate, Disodium Lauramido PEG-5 Sulfosuccinate, Disodium Laureth-6 Sulfosuccinate, Disodium Laureth-9 Sulfosuccinate, Disodium Laureth-12 Sulfosuccinate, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleth-3 Sulfosuccinate, Disodium Oleyl Sulfosuccinate, Disodium Palmitamido PEG-2 Sulfosuccinate, Disodium Palmitoleamido PEG-2 Sulfosuccinate, Disodium PEG-4 Cocamido MIPA-Sulfo-succinate, Disodium PEG-5 Laurylcitrate Sulfosuccinate, Disodium PEG-8 Palm Glycerides Sulfosuccinate, Disodium Ricinoleamido MEA-Sulfosuccinate, Disodium Sitostereth-14 Sulfosuccinate, Disodium Stearamido MEA-Sulfosuccinate, Disodium Stearyl Sulfosuccinamate, Disodium Stearyl Sulfosuccinate, Disodium Tallamido MEA-Sulfosuccinate, Disodium Tallowamido MEA-Sulfo-succinate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Wheat Germamido MEA-Sulfosuccinate, Disodium Wheat Germamido PEG-2 Sulfosuccinate, Di-TEA-Oleamido PEG-2 Sulfosuccinate, Ditridecyl Sodium Sulfosuccinate, Sodium Bisglycol Ricinosulfosuccinate, Sodium/MEA Laureth-2 Sulfosuccinate und Tetrasodium Dicarboxyethyl Stearyl Sulfosuccinamate. Noch ein weiteres geeignetes Sulfosuccinamat ist Dinatrium-C₁₆₋₁₈-alkoxypropylen-sulfosuccinamat.

Bevorzugte anionische Sulfobernsteinsäuretenside sind Imidosuccinat, Mono-Na-sulfobernsteinsäure-di-isobutylester (Monawet® MB 45), Mono-Na-sulfobernsteinsäure-di-octylester (Monawet® MO-84 R2W, Rewopol® SB DO 75), Mono-Na-sulfobernsteinsäure-di-tridecylester (Monawet® MT 70), Fettalkoholpolyglykolsulfosuccinat-Na-NH₄-Salz (Sulfosuccinat S-2), Di-Nasulfobernstein-säure-mono-C_{12/14}-3EO-ester (Texapon® SB-3), Natruimsulfobernsteinsäurediisooctylester (Texin® DOS 75) und Di-Na-Sulfobernsteinsäure-mono-C_{12/18}-ester (Texin® 128-P), insbesondere der mit der erfindungsgemäßen ternären Tensidkombination hinsichtlich des Ablauf- und/oder Trocknungsverhaltens synergistisch zusammenwirkende Mono-Na-sulfobernsteinsäure-di-octylester.

In einer besonderen Ausführungsform enthält das erfindungsgemäße Mittel als anionische Sulfobernsteinsäuretenside ein oder mehrere Sulfosuccinate, Sulfosuccinamate und/oder Sulfosuccinamide, vorzugsweise Sulfosuccinate und/oder Sulfosuccinamate, insbesondere Sulfosuccinate, in einer Menge von üblicherweise 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%.

In einer bevorzugten Ausführungsform der vorliegenden Erfindung beträgt der Anteil des anionischen Tensids 5 bis 20 Gew.-%, besonders bevorzugt 8 bis 16 Gew.-%, bezogen auf das Gesamtgewicht des Reinigungsmittels, insbesondere des Handgeschirrspülmittels. In einer besonders bevorzugten Ausführungsform enthält das erfindungsgemäße Reinigungsmittel 10 Gew.-% anionisches Tensid. Vorzugsweise handelt es sich bei dem anionischen Tensid um ein Fettalkoholethersulfat. Bevorzugte Fettalkoholethersulfate sind oben beschrieben.

Zu den Amphotensiden (amphoteren Tensiden, zwitterionischen Tensiden), die erfindungsgemäß eingesetzt werden können, zählen Alkylamidoalkylamine, alkylsubstituierte Aminosäuren, acylierte Aminosäuren bzw. Biotenside, von denen die Betaine im Rahmen der erfindungsgemäßen Lehre bevorzugt werden.

Geeignete Betaine, welche vor allem in manuellen Geschirrspülmitteln Einsatz finden, sind die Alkylbetaine, die Alkylamidobetaine, die Imidazoliniumbetaine, die Sulfobetaine (INCI Sultaines) sowie die Phosphobetaine und genügen vorzugsweise Formel I,

R¹-[CO-X-(CH₂)ₙ]ₓ-N⁺(R²)(R³)-(CH₂)ₘ-[CH(OH)-CH₂]_{y}Y⁻ (I)

in der
- R¹: ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- X: NH, NR⁴ mit dem C₁₋₄-Alkylrest R⁴, O oder S,
- n: eine Zahl von 1 bis 10, vorzugsweise 2 bis 5, insbesondere 3,
- x: 0 oder 1, vorzugsweise 1,
- R², R³: unabhängig voneinander ein C₁₋₄-Alkylrest, ggf. hydroxysubstituiert wie z.B. ein Hydroxyethylrest, insbesondere aber ein Methylrest,
- m: eine Zahl von 1 bis 4, insbesondere 1, 2 oder 3,
- y: 0 oder 1 und
- Y: COO, SO₃, OPO(OR⁵)O oder P(O)(OR⁵)O, wobei R⁵ ein Wasserstoffatom H oder ein C₁₋₄-Alkylrest ist.

Die Alkyl- und Alkylamidobetaine, Betaine der Formel I mit einer Carboxylatgruppe (Y⁻ = COO⁻), heißen auch Carbobetaine.

Bevorzugte Betaine sind die Alkylbetaine der Formel (la), die Alkylamidobetaine der Formel (Ib), die Sulfobetaine der Formel (Ic) und die Amidosulfobetaine der Formel (Id),

R¹-N⁺(CH₃)₂-CH₂COO⁻ (Ia)

R¹-CO-NH-(CH₂)₃-N⁺(CH₃)₂-CH₂COO⁻ (Ib)

R⁺-N⁺(CH₃)₂-CH₂CH(OH)CH₂SO₃⁻ (Ic)

R¹-CO-NH-(CH₂)₃-N⁺(CH₃)₂-CH₂CH(OH)CH₂SO₃⁻ (Id)

in denen R¹ die gleiche Bedeutung wie in Formel I hat.

Besonders bevorzugte Betaine sind die Carbobetaine, insbesondere die Carbobetaine der Formel (la) und (Ib), äußerst bevorzugt die Alkylamidobetaine der Formel (Ib).

Beispiele geeigneter Betaine und Sulfobetaine sind die folgenden gemäß INCI benannten Verbindungen: Almondamidopropyl Betaine, Apricotamidopropyl Betaine, Avocadamidopropyl Betaine, Babassuamidopropyl Betaine, Behenamidopropyl Betaine, Behenyl Betaine, Betaine, Canolamidopropyl Betaine, Capryl/Capramidopropyl Betaine, Carnitine, Cetyl Betaine, Cocamidoethyl Betaine, Cocamidopropyl Betaine, Cocamidopropyl Hydroxysultaine, Coco-Betaine, Coco-Hydroxy-sultaine, Coco/Oleamidopropyl Betaine, Coco-Sultaine, Decyl Betaine, Dihydroxyethyl Oleyl Glycinate, Dihydroxyethyl Soy Glycinate, Dihydroxyethyl Stearyl Glycinate, Dihydroxyethyl Tallow Glycinate, Dimethicone Propyl PG-Betaine, Erucamidopropyl Hydroxysultaine, Hydrogenated Tallow Betaine, Isostearamidopropyl Betaine, Lauramidopropyl Betaine, Lauryl Betaine, Lauryl Hydroxysultaine, Lauryl Sultaine, Milkamidopropyl Betaine, Minkamidopropyl Betaine, Myristamidopropyl Betaine, Myristyl Betaine, Oleamidopropyl Betaine, Oleamidopropyl Hydroxysultaine, Oleyl Betaine, Olivamidopropyl Betaine, Palmamidopropyl Betaine, Palmitamidopropyl Betaine, Palmitoyl Carnitine, Palm Kernelamidopropyl Betaine, Polytetrafluoroethylene Acetoxypropyl Betaine, Ricinoleamidopropyl Betaine, Sesamidopropyl Betaine, Soyamidopropyl Betaine, Stearamidopropyl Betaine, Stearyl Betaine, Tallowamidopropyl Betaine, Tallowamidopropyl Hydroxysultaine, Tallow Betaine, Tallow Dihydroxyethyl Betaine, Undecylenamidopropyl Betaine und Wheat Germamidopropyl Betaine. Ein bevorzugtes Betain ist beispielsweise Cocamidopropyl Betaine (Cocoamidopropylbetain).

Die Alkylamidoalkylamine (INCI Alkylamido Alkylamines) sind Amphotenside der Formel (III),

R⁹-CO-NR¹⁰-(CH₂)ᵢ-N(R¹¹)-(CH₂CH₂O)ⱼ-(CH₂)ₖ-[CH(OH)]ᵢ-CH₂-Z-OM (III)

in der
- R⁹: ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- R¹⁰: ein Wasserstoffatom H oder ein C₁₋₄-Alkylrest, vorzugsweise H,
- i: eine Zahl von 1 bis 10, vorzugsweise 2 bis 5, insbesondere 2 oder 3,
- R¹¹: ein Wasserstoffatom H oder CH₂COOM (zu M s.u.),
- j: eine Zahl von 1 bis 4, vorzugsweise 1 oder 2, insbesondere 1,
- k: eine Zahl von 0 bis 4, vorzugsweise 0 oder 1,
- l: 0 oder 1, wobei k = 1 ist, wenn I = 1 ist,
- Z: CO, SO₂, OPO(OR¹²) oder P(O)(OR¹²), wobei R¹² ein C₁₋₄-Alkylrest oder M (s.u.) ist, und
- M: ein Wasserstoff, ein Alkalimetall, ein Erdalkalimetall oder ein protoniertes Alkanolamin, z.B. protoniertes Mono-, Di- oder Triethanolamin, ist.

Bevorzugte Vertreter genügen den Formeln IIIa bis IIId,

R⁹-CO-NH-(CH₂)₂-N(R¹¹)-CH₂CH₂O-CH₂-COOM (IIIa)

R⁹-CO-NH-(CH₂)₂-N(R¹¹)-CH₂CH₂O-CH₂CH₂-COOM (IIIb)

R⁹-CO-NH-(CH₂)₂-N(R¹¹)-CH₂CH₂O-CH₂CH(OH)CH₂-SO₃M (IIIc)

R⁹-CO-NH-(CH₂)₂-N(R¹¹)-CH₂CH₂O-CH₂CH(OH)CH₂-OPO₃HM (IIId)

in denen R¹¹ und M die gleiche Bedeutung wie in Formel (III) haben.

Beispielhafte Alkylamidoalkylamine sind die folgenden gemäß INCI benannten Verbindungen: Cocoamphodipropionic Acid, Cocobetainamido Amphopropionate, DEA-Cocoamphodipropionate, Disodium Caproamphodiacetate, Disodium Caproamphodipropionate, Disodium Capryloamphodiacetate, Disodium Capryloamphodipropionate, Disodium Cocoamphocarboxyethylhydroxypropylsulfonate, Disodium Cocoamphodiacetate, Disodium Cocoamphodipropionate, Disodium Isostearoamphodiacetate, Disodium Isostearoamphodipropionate, Disodium Laureth-5 Carboxyamphodiacetate, Disodium Lauroamphodiacetate, Disodium Lauroamphodipropionate, Disodium Oleoamphodipropionate, Disodium PPG-2-Isodeceth-7 Carboxyamphodiacetate, Disodium Stearoamphodiacetate, Disodium Tallowamphodiacetate, Disodium Wheatgermamphodiacetate, Lauroamphodipropionic Acid, Quaternium-85, Sodium Caproamphoacetate, Sodium Caproamphohydroxypropylsulfonate, Sodium Caproamphopropionate, Sodium Capryloamphoacetate, Sodium Capryloamphohydroxypropylsulfonate, Sodium Capryloamphopropionate, Sodium Cocoamphoacetate, Sodium Cocoamphohydroxypropylsulfonate, Sodium Cocoamphopropionate, Sodium Cornamphopropionate, Sodium Isostearoamphoacetate, Sodium Isostearoamphopropionate, Sodium Lauroamphoacetate, Sodium Lauroamphohydroxypropylsulfonate, Sodium Lauroampho PG-Acetate Phosphate, Sodium Lauroamphopropionate, Sodium Myristoamphacetate, Sodium Oleoamphoacetate, Sodium Oleoamphohydroxypropylsulfonate, Sodium Oleoamphopropionate, Sodium Ricinoleoamphoacetate, Sodium Stearoamphoacetate, Sodium Stearoamphohydroxypropylsulfonate, Sodium Stearoamphopropionate, Sodium Tallamphopropionate, Sodium Tallowamphoacetate, Sodium Undecylenoamphoacetate, Sodium Undecylenoamphopropionate, Sodium Wheat Germamphoacetate und Trisodium Lauroampho PG-Acetate Chloride Phosphate.

Erfindungsgemäß bevorzugte alkylsubstituierte Aminosäuren (INCI Alkyl-Substituted Amino Acids) sind monoalkylsubstituierte Aminosäuren gemäß Formel (IV),

R¹³-NH-CH(R¹⁴HCH₂)ᵤ-COOM' (IV)

in der
- R¹³: ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- R¹⁴: ein Wasserstoffatom H oder ein C₁₋₄-Alkylrest, vorzugsweise H,
- u: eine Zahl von 0 bis 4, vorzugsweise 0 oder 1, insbesondere 1, und
- M': ein Wasserstoff, ein Alkalimetall, ein Erdalkalimetall oder ein protoniertes Alkanolamin, z.B. protoniertes Mono-, Di- oder Triethanolamin, ist,
alkylsubstituierte Iminosäuren gemäß Formel (V),

R¹⁵-N-[(CH₂)ᵥ-COOM"]₂ (V)

in der
- R¹⁵: ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- v: eine Zahl von 1 bis 5, vorzugsweise 2 oder 3, insbesondere 2, und
- M": ein Wasserstoff, ein Alkalimetall, ein Erdalkalimetall oder ein protoniertes Alkanolamin, z.B. protoniertes Mono-, Di- oder Triethanolamin, wobei M" in den beiden Carboxygruppen die gleiche oder zwei verschiedene Bedeutungen haben kann, z.B. Wasserstoff und Natrium oder zweimal Natrium sein kann, ist,
und mono- oder dialkylsubstituierte natürliche Aminosäuren gemäß Formel (VI),

R¹⁶-N(R¹⁷)-CH(R¹⁸)-COOM"' (VI)

in der
- R¹⁶: ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- R¹⁷: ein Wasserstoffatom oder ein C₁₋₄-Alkylrest, ggf. hydroxy- oder aminsubstituiert, z.B. ein Methyl-, Ethyl-, Hydroxyethyl- oder Aminpropylrest,
- R¹⁸: den Rest einer der 20 natürlichen α-Aminosäuren H₂NCH(R¹⁸)COOH, und
- M'": ein Wasserstoff, ein Alkalimetall, ein Erdalkalimetall oder ein protoniertes Alkanolamin, z.B. protoniertes Mono-, Di- oder Triethanolamin, ist.

Besonders bevorzugte alkylsubstituierte Aminosäuren sind die Aminopropionate gemäß Formel (IVa),

R¹³-NH-CH₂CH₂COOM' (IVa)

in der R¹³ und M' die gleiche Bedeutung wie in Formel (IV) haben.

Beispielhafte alkylsubstituierte Aminosäuren sind die folgenden gemäß INCI benannten Verbindungen: Aminopropyl Laurylglutamine, Cocaminobutyric Acid, Cocaminopropionic Acid, DEA-Lauraminopropionate, Disodium Cocaminopropyl Iminodiacetate, Disodium Dicarboxyethyl Cocopropylenediamine, Disodium Lauriminodipropionate, Disodium Steariminodipropionate, Disodium Tallowiminodipropionate, Lauraminopropionic Acid, Lauryl Aminopropylglycine, Lauryl Diethylenediaminoglycine, Myristaminopropionic Acid, Sodium C12-15 Alkoxypropyl Iminodipropionate, Sodium Cocaminopropionate, Sodium Lauraminopropionate, Sodium Lauriminodipropionate, Sodium Lauroyl Methylaminopropionate, TEA-Lauraminopropionate und TEA-Myristaminopropionate.

Acylierte Aminosäuren sind Aminosäuren, insbesondere die 20 natürlichen α-Aminosäuren, die am Aminostickstoffatom den Acylrest R¹⁹CO einer gesättigten oder ungesättigen Fettsäure R¹⁹COOH tragen, wobei R¹⁹ ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest ist. Die acylierten Aminosäuren können auch als Alkalimetallsalz, Erdalkalimetallsalz oder Alkanolammoniumsalz, z.B. Mono-, Di- oder Triethanolammoniumsalz, eingesetzt werden. Beispielhafte acylierte Aminosäuren sind die gemäß INCI unter Amino Acids zusammengefassten Acylderivate, z.B. Sodium Cocoyl Glutamate, Lauroyl Glutamic Acid, Capryloyl Glycine oder Myristoyl Methylalanine.

In einer besonderen Ausführungsform der Erfindung wird eine Kombination aus zwei oder mehr verschiedenen Amphotensiden, insbesondere eine binäre Amphotensidkombination eingesetzt. Die Amphotensidkombination enthält vorzugsweise mindestens ein Betain, insbesondere mindestens ein Alkylamidobetain, besonders bevorzugt Cocoamidopropylbetain.

Weiterhin enthält die Amphotensidkombination vorzugsweise mindestens ein amphoteres Tensid aus der Gruppe umfassend Natriumcarboxyethylkokosphosphoethylimidazolin (Phosphoteric® TC-6), C_{8/10}-Amidopropylbetain (INCI Capryl/Capramidopropyl Betaine; Tego® Betaine 810), N-2-Hydroxyethyl-N-carboxymethyl-fettsäureamido-ethylamin-Na (Rewoteric® AMV) und N-Capryl/Caprin-amidoethyl-N-ethylether-propionat-Na (Rewoteric® AMVSF) sowie das Betain 3-(3-Cocoamido-propyl)-dimethylammonium-2-hydroxypropansulfonat (INCI Sultaine; Rewoteric® AM CAS) und das Alkylamidoalkylamin N-[N'(N"-2-Hydroxyethyl-N"-carboxyethylaminoethyl)-essigsäureamido]-N,N-dimethyl-N-cocos-ammoniumbetain (Rewoteric® QAM 50), insbesondere zusammen mit Cocoamidopropylbetain.

Das erfindungsgemäße Mittel enthält in einer bevorzugten Ausführungsform ein oder mehrere Amphotenside, insbesondere Betaine, in einer Menge von 0,5 bis 15 Gew.-%, vorzugsweise 1 bis 10 Gew.-%, insbesondere 2 bis 8 Gew.-%. In einer ganz besonders bevorzugten Ausführungsform enthält das Mittel 4 Gew.-% Amphotenside (insbesondere Betain). Bevorzugte Betaine wurden oben beschrieben. Ein ganz besonders bevorzugtes Betain ist Cocoamidopropylbetain.

In einer bevorzugten Ausführungsform enthält das erfindungsgemäße Mittel eine Tensidkombination aus mindestens einem anionischen Tensid und mindestens einem, amphoteren Tensid. Dabei liegen diese Tenside vorzugsweise in einem Massenverhältnis von 10:1 bis 1:5, vorzugsweise von 5:1 bis 2:1, insbesondere bevorzugt von 4:1 vor. Vorzugsweise umfasst das mindestens eine anionische Tensid mindestens ein Alkylethersulfat und das mindestens eine amphotere Tensid mindestens ein Betain-Tensid.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO, 4 EO oder 7 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf

(narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Insbesondere bevorzugt enthält das Handgeschirrspülmittel einen C₁₂₋₁₈-Fettalkohol mit 7 EO oder einen C₁₃₋₁₅-Oxoalkohol mit 7 EO als nichtionisches Tensid.

In einer weiteren bevorzugten Ausführungsform des flüssigen Reinigungsmittels, insbesondere des Handgeschirrspülmittels, ist bevorzugt als weiteres Tensid ein nichtionisches Tensid, vorzugsweise ein Fettalkoholpolyglykolether enthalten, vorzugsweise in Mengen von 0,001 bis 10 Gew.-%, besonders bevorzugt 0,01 bis 6 Gew.-%, weiter bevorzugt 0,1 bis 4 Gew.-%, insbesondere 0,2 bis 2 Gew.-%., jeweils bezogen auf das gesamte Handgeschirrspülmittel.

Diese nichtionische Tenside weisen in Kombination mit einem Aminoxid eine gute Reinigungsleistung an fettverschmutzten harten Oberflächen, wie beispielsweise Geschirr, auf.

Nichtionische Tenside im Rahmen der Erfindung sind Alkoxylate, aber auch Alkylphenolpolyglykolether, endgruppenverschlossene Polyglykolether, Mischether und Hydroxymischether und Fettsäurepolyglykolester. Ebenfalls geeignet sind Blockpolymere aus Ethylenoxid und Propylenoxid sowie Fettsäurealkanolamide und Fettsäurepolyglykolether. Wichtige Klassen erfindungsgemäßer nichtionischer Tenside sind weiterhin die Aminoxide und die Zuckertenside, insbesondere die Alkylpolyglucoside.

Zu den erfindungsgemäß geeigneten Aminoxiden gehören Alkylaminoxide, insbesondere Alkyldimethylaminoxide, Alkylamidoaminoxide und Alkoxyalkylaminoxide. Bevorzugte Aminoxide genügen Formel II,

R⁶R⁷R⁸N⁺-O⁻ (II)

R⁶-[CO-NH-(CH₂)_{w}]_{z}-N⁺(R⁷)(R⁸)-O⁻ (II)

in der
- R⁶: ein gesättiger oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest, der in den Alkylamidoaminoxiden über eine Carbonylamidoalkylengruppe -CO-NH-(CH₂)_{z}- und in den Alkoxyalkylaminoxiden über eine Oxaalkylengruppe -O-(CH₂)_{z}- an das Stickstoffatom N gebunden ist, wobei z jeweils für eine Zahl von 1 bis 10, vorzugsweise 2 bis 5, insbesondere 3,
- R⁷, R⁸: unabhängig voneinander ein C₁₋₄-Alkylrest, ggf. hydroxysubstituiert wie z.B. ein Hydroxyethylrest, insbesondere ein Methylrest, ist.

Beispiele geeigneter Aminoxide sind die folgenden gemäß INCI benannten Verbindungen: Almondamidopropylamine Oxide, Babassuamidopropylamine Oxide, Behenamine Oxide, Cocamidopropyl Amine Oxide, Cocamidopropylamine Oxide, Cocamine Oxide, Coco-Morpholine Oxide, Decylamine Oxide, Decyltetradecylamine Oxide, Diaminopyrimidine Oxide, Dihydroxyethyl C8-10 Alkoxypropylamine Oxide, Dihydroxyethyl C9-11 Alkoxypropylamine Oxide, Dihydroxyethyl C12-15 Alkoxypropylamine Oxide, Dihydroxyethyl Cocamine Oxide, Dihydroxyethyl Lauramine Oxide, Dihydroxyethyl Stearamine Oxide, Dihydroxyethyl Tallowamine Oxide, Hydrogenated Palm Kernel Amine Oxide, Hydrogenated Tallowamine Oxide, Hydroxyethyl Hydroxypropyl C12-15 Alkoxypropylamine Oxide, Isostearamidopropylamine Oxide, Isostearamidopropyl Morpholine Oxide, Lauramidopropylamine Oxide, Lauramine Oxide, Methyl Morpholine Oxide, Milkamidopropyl Amine Oxide, Minkamidopropylamine Oxide, Myristamidopropylamine Oxide, Myristamine Oxide, Myristyl/Cetyl Amine Oxide, Oleamidopropylamine Oxide, Oleamine Oxide, Olivamidopropylamine Oxide, Palmitamidopropylamine Oxide, Palmitamine Oxide, PEG-3 Lauramine Oxide, Potassium Dihydroxyethyl Cocamine Oxide Phosphate, Potassium Trisphosphonomethylamine Oxide, Sesamidopropylamine Oxide, Soyamidopropylamine Oxide, Stearamidopropylamine Oxide, Stearamine Oxide, Tallowamidopropylamine Oxide, Tallowamine Oxide, Undecylenamidopropylamine Oxide und Wheat Germamidopropylamine Oxide. Bevorzugte Aminoxide sind beispielsweise Cocamidopropylamine Oxide (Cocoamidopropylaminoxid), aber auch N-Kokosalkyl-N,N-dimethylaminoxid, N-Talgalkyl-N,N-dihydroxyethylaminoxid, Myristylcetyldimethylaminoxid oder Lauryldimethylaminoxid.

Der Gehalt an Aminoxid beträgt in dem erfindungsgemäßen Mittel bevorzugt 1 bis 15 Gew.-% und vorzugsweise 2 bis 10 Gew.-%, jeweils bezogen auf das gesamte Mittel.

Zuckertenside sind bekannte oberflächenaktive Verbindungen, zu denen beispielsweise die Zuckertensidklassen der Alkylglucoseester, Aldobionamide, Gluconamide (Zuckersäureamide), Glycerinamide, Glyceringlykolipide, Polyhydroxyfettsäureamidzuckertenside (Zuckeramide) und Alkylpolyglykoside zählen. Im Rahmen der erfindungsgemäßen Lehre bevorzugte Zuckertenside sind die Alkylpolyglykoside und die Zuckeramide sowie deren Derivate, insbesondere ihre Ether und Ester. Bei den Ethern handelt es sich um die Produkte der Reaktion einer oder mehrerer, vorzugsweise einer, Zuckerhydroxygruppe mit einer eine oder mehrere Hydroxygruppen enthaltenden Verbindung, beispielsweise C₁₋₂₂-Alkoholen oder Glykolen wie Ethylen- und/oder Propylenglykol, wobei die Zuckerhydroxygruppe auch Polyethylenglykol- und/oder Polypropylenglykolreste tragen kann. Die Ester sind die Reaktionsprodukte einer oder mehrerer, vorzugsweise einer, Zuckerhydroxygruppe mit einer Carbonsäure, insbesondere einer C₆₋₂₂-Fettsäure.

Besonders bevorzugte Zuckeramide genügen der Formel R'C(O)N(R")[Z], in der R' für einen linearen oder verzweigten, gesättigten oder ungesättigten Acylrest, vorzugsweise einen linearen ungesättigten Acylrest, mit 5 bis 21, vorzugsweise 5 bis 17, insbesondere 7 bis 15, besonders bevorzugt 7 bis 13 Kohlenstoffatomen, R" für einen linearen oder verzweigten, gesättigten oder ungesättigten Alkylrest, vorzugsweise einen linearen ungesättigten Alkylrest, mit 6 bis 22, vorzugsweise 6 bis 18, insbesondere 8 bis 16, besonders bevorzugt 8 bis 14 Kohlenstoffatomen, einen C₁₋₅-Alkylrest, insbesondere einen Methyl-, Ethyl-, Propyl-, Isopropyl-, n-Butyl-, Isobutyl-, tert-Butyl- oder n-Pentylrest, oder Wasserstoff und Z für einen Zuckerrest, d.h. einen Monosaccharidrest, stehen. Besonders bevorzugte Zuckeramide sind die Amide der Glucose, die Glucamide, beispielsweise Lauroyl-methyl-glucamid.

Die Alkylpolyglykoside (APG) sind im Rahmen der erfindungsgemäßen Lehre besonders bevorzugte Zuckertenside und genügen vorzugsweise der allgemeinen Formel RⁱO(AO)ₐ[G]ₓ, in der R' für einen linearen oder verzweigten, gesättigten oder ungesättigten Alkylrest mit 6 bis 22, vorzugsweise 6 bis 18, insbesondere 8 bis 16, besonders bevorzugt 8 bis 14 Kohlenstoffatomen, [G] für einen glykosidisch verknüpften Zuckerrest und x für eine Zahl von 1 bis 10 sowie AO für eine Alkylenoxygruppe, z.B. eine Ethylenoxy- oder Propylenoxygruppe, und a für den mittleren Alkoxylierungsgrad von 0 bis 20 stehen. Hierbei kann die Gruppe (AO)ₐ auch verschiedene Alkylenoxyeinheiten enthalten, z.B. Ethylenoxy- oder Propylenoxyeinheiten, wobei es sich dann bei a um den mittleren Gesamtalkoxylierungsgrad, d.h. die Summe aus Ethoxylierungs- und Propoxylierungsgrad, handelt. Soweit nachfolgend nicht näher bzw. anders ausgeführt, handelt es sich bei den Alkylresten R' der APG um lineare ungesättigte Reste mit der angegebenen Zahl an Kohlenstoffatomen.

APG sind nichtionische Tenside und stellen bekannte Stoffe dar, die nach den einschlägigen Verfahren der präparativen organischen Chemie erhalten werden können. Die Indexzahl x gibt den Oligomerisierungsgrad (DP-Grad) an, d.h. die Verteilung von Mono- und Oligoglykosiden, und steht für eine Zahl zwischen 1 und 10. Während x in einer gegebenen Verbindung stets ganzzahlig sein muss und hier vor allem die Werte x = 1 bis 6 annehmen kann, ist der Wert x für ein bestimmtes Alkylglykosid eine analytisch ermittelte rechnerische Größe, die meistens eine gebrochene Zahl darstellt. Vorzugsweise werden Alkylglykoside mit einem mittleren Oligomerisierungsgrad x von 1,1 bis 3,0 eingesetzt. Aus anwendungstechnischer Sicht sind solche Alkylglykoside bevorzugt, deren Oligomerisierungsgrad kleiner als 1,7 ist und insbesondere zwischen 1,2 und 1,6 liegt. Als glykosidischer Zucker wird vorzugsweise Xylose, insbesondere aber Glucose verwendet.

Der Alkyl- bzw. Alkenylrest R' kann sich von primären Alkoholen mit 8 bis 18, vorzugsweise 8 bis 14 Kohlenstoffatomen ableiten. Typische Beispiele sind Capronalkohol, Caprylalkohol, Caprinalkohol und Undecylalkohol sowie deren technische Gemische, wie sie beispielsweise im Verlauf der Hydrierung von technischen Fettsäuremethylestern oder im Verlauf der Hydrierung von Aldehyden aus der RoELENschen Oxosynthese anfallen.

Vorzugsweise leitet sich der Alkyl- bzw. Alkenylrest R' aber von Laurylalkohol, Myristylalkohol, Cetylalkohol, Palmoleylalkohol, Stearylalkohol, Isostearylalkohol oder Oleylalkohol ab. Weiterhin sind Elaidylalkohol, Petroselinylalkohol, Arachidylalkohol, Gadoleylalkohol, Behenylalkohol, Erucylalkohol sowie deren technische Gemische zu nennen.

Besonders bevorzugte APG sind nicht alkoxyliert (a = 0) und genügen Formel RO[G]ₓ, in der R wie zuvor für einen linearen oder verzweigten, gesättigten oder ungesättigten Alkylrest mit 4 bis 22 Kohlenstoffatomen, [G] für einen glykosidisch verknüpften Zuckerrest, vorzugsweise Glucoserest, und x für eine Zahl von 1 bis 10, bevorzugt 1,1 bis 3, insbesondere 1,2 bis 1,6, stehen. Dementsprechend bevorzugte Alkylpolyglykoside sind beispielsweise C₈₋₁₀- und ein C₁₂₋₁₄-Alkylpolyglucosid mit einem DP-Grad von 1,4 oder 1,5, insbesondere C₈₋₁₀- Alkyl-1,5-glucosid und C₁₂₋₁₄-Alkyl-1,4-glucosid.

Das erfindungsgemäße Mittel kann zusätzlich ein oder mehrere kationische Tenside (Kationtenside; INCI Quaternary Ammonium Compounds) enthalten, üblicherweise in einer Menge von 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%.

Bevorzugte kationische Tenside sind die quaternären oberflächenaktiven Verbindungen, insbesondere mit einer Ammonium-, Sulfonium-, Phosphonium-, Jodonium- oder Arsoniumgruppe, die auch als antimikrobielle Wirkstoffe bekannt sind. Durch den Einsatz von quaternären oberflächenaktiven Verbindungen mit antimikrobieller Wirkung kann das Mittel mit einer antimikrobiellen Wirkung ausgestaltet werden bzw. dessen gegebenenfalls aufgrund anderer Inhaltsstoffe bereits vorhandene antimikrobielle Wirkung verbessert werden.

Besonders bevorzugte kationische Tenside sind die quaternären Ammoniumverbindungen (QAV; INCI Quaternary Ammonium Compounds) gemäß der allgemeinen Formel (R^{I})(R^{II})(R^{III})(R^{IV})N⁺X⁻, in der R^{I} bis R^{IV} gleiche oder verschiedene C₁₋₂₂-Alkylreste, C₇₋₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere 12 bis 16, C-Atomen auf.

QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielsweise Benzalkoniumchlorid (N-Alkyl-N,N-dimethyl-benzylammoniumchlorid, CAS No. 8001-54-5), Benzalkon B (m,p-Dichlorbenzyl-dimethyl-C₁₂-alkylammoniumchlorid, CAS No. 58390-78-6), Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid, CAS No. 57-09-0), Benzetoniumchlorid (N,N-Dimethyl-N-[2-[2-[p-(1,1,3,3-tetramethylbutyl)phenoxy]-ethoxy]ethyl]-benzylammoniumchlorid, CAS No. 121-54-0), Dialkyldimethylammoniumchloride wie Di-n-decyl-dimethyl-ammoniumchlorid (CAS No. 7173-51-5-5), Didecyldimethylammoniumbromid (CAS No. 2390-68-3), Dioctyl-dimethyl-ammoniumchlorid, 1-Cetylpyridiniumchlorid (CAS No. 123-03-5) und Thiazolinjodid (CAS No. 15764-48-1) sowie deren Mischungen. Bevorzugte QAV sind die Benzalkoniumchloride mit C₈-C₁₈-Alkylresten, insbesondere C₁₂-C₁₄-Alkyl-benzyldimethylammoniumchlorid. Eine besonders bevorzugte QAV ist das Kokospentaethoxymethylammoniummethosulfat (INCI PEG-5 Cocomonium Methosulfate; Rewoquat® CPEM).

Zur Vermeidung möglicher Inkompatibilitäten der kationischen Tenside mit den anionischen Tensiden können aniontensidverträgliches und/oder möglichst wenig kationisches Tensid eingesetzt oder in einer besonderen Ausführungsform der Erfindung gänzlich auf kationische Tenside verzichtet. In einer bevorzugten Ausführungsform ist das erfindungsgemäße Mittel frei von kationischen und nichtionischen Tensiden.

Zur Stabilisierung der eingesetzten Amylase und gegebenenfalls weiterer enthaltener Enzyme ist es vorteilhaft, wenn dem erfindungsgemäßen Reinigungsmittel ein Enzymstabilisator zugefügt wird. Aus dem Stand der Technik sind verschiedene geeignete Stabilisatoren bekannt, etwa Saccharide, Polyole, insbesondere Propylenglykol oder Glycerin, weiterhin Borax, Borsäuren, Boronsäuren oder deren Salze oder Ester, insbesondere Derivate mit aromatischen Gruppen, etwa ortho-, meta- oder para-substituierte Phenylboronsäuren, oder auch Calciumsalze wie Calciumformiat, Calciumacetat oder Calciumpropionat.

Als besonders vorteilhaft hat sich jedoch der Einsatz von Kaliumsalzen erwiesen. Hierbei können sowohl anorganische als auch organische Kaliumsalze als Quelle der Kaliumionen verwendet werden. Bevorzugte Kaliumsalze sind farblos. Unter den anorganischen Kaliumsalze sind solche aus der Gruppe der wasserlöslichen Halogenide, Sulfate, Sulfite, Carbonate, Hydrogencarbonate, Nitrate, Nitrite, Phosphate und/oder Oxide bevorzugt, insbesondere Halogenide und Sulfate. Bei den organischen Kaliumsalzen, die erfindungsgemäß als Quelle für die Kaliumionen herangezogen werden können, handelt es sich insbesondere um wasserlösliche Kaliumsalze der Carbonsäuren. Vorzugsweise sind die Salze ausgewählt aus der Gruppe bestehend aus Formiat, Acetat, Propionat, Citrat, Malat, Tartrat, Succinat, Malonat, Oxalat, Lactat sowie Gemischen derselben.

Besonders vorteilhaft sind Kaliumchlorid, Kaliumsulfat, Kaliumformiat, Kaliumacetat, Kaliumpropionat und Kaliumlactat sowie Gemische davon als Kaliumionenquelle vorgesehen. Kaliumacetat und Kaliumchlorid sind dabei ganz besonders bevorzugt. Das erfindungsgemäße Reinigungsmittel enthält daher Kaliumionen, deren Anteil an der Gesamtzusammensetzung 0,025 bis 0,25 Gew.-% beträgt, vorzugsweise 0,05 bis 0,125 Gew.-%. Vorteilhafterweise sind in einer Ausführungsform der Erfindung in dem erfindungsgemäßen Mittel keine Buildersubstanzen vorhanden, welche Calcium-fällende Eigenschaften aufweisen. Dementsprechend wird ein Mittel bevorzugt, welches insbesondere keine Carbonat-haltigen Salze enthält.

Der Einsatz anderer Buildersubstanzen, vorzugsweise wasserlöslicher Buildersubstanzen kann hingegen von Vorteil sein.

Organische Buildersubstanzen, welche in Handgeschirrspülmittel vorhanden sein können, sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), Methylglycindiessigsäure (MGDA) und deren Abkömmlinge sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Als Gerüststoffe sind weiter polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g / mol.

Geeignete Polymere sind insbesondere Polyacrylate, die bevorzugt eine Molekülmasse von 1.000 bis 15.000 g / mol aufweisen. Aufgrund ihrer überlegenen Löslichkeit können aus dieser Gruppe wiederum die kurzkettigen Polyacrylate, die Molmassen von 1.000 bis 10.000 g / mol, und besonders bevorzugt von 1.000 bis 5.000 g / mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten.

Bevorzugt werden allerdings lösliche Gerüststoffe, wie beispielsweise Citronensäure, oder Acrylpolymere mit einer Molmasse von 1.000 bis 5.000 g / mol in den flüssigen Handgeschirrspülmitteln eingesetzt.

Der Wassergehalt des bevorzugten flüssigen und wässerigen Handgeschirrspülmittels beträgt üblicherweise 15 bis 90 Gew.-%, vorzugsweise 20 bis 85 Gew.-%, insbesondere 30 bis 80 Gew.-%. Das erfindungsgemäße Mittel kann zusätzlich ein oder mehrere wasserlösliche organische Lösungsmittel enthalten, üblicherweise in einer Menge von 0,1 bis 30 Gew.-%, vorzugsweise 1 bis 20 Gew.-%, insbesondere 2 bis 15 Gew.-%, besonders bevorzugt 3 bis 12 Gew.-%, äußerst bevorzugt 4 bis 8 Gew.-%.

Das Lösungsmittel wird im Rahmen der erfindungsgemäßen Lehre nach Bedarf insbesondere als Hydrotropikum, Viskositätsregulator und/oder zusätzlicher Kältestabilisator eingesetzt. Es wirkt lösungsvermittelnd insbesondere für Tenside und Elektrolyt sowie Parfüm und Farbstoff und trägt so zu deren Einarbeitung bei, verhindert die Ausbildung flüssigkristalliner Phasen und hat Anteil an der Bildung klarer Produkte. Die Viskosität des erfindungsgemäßen Mittels verringert sich mit zunehmender Lösungsmittelmenge. Zuviel Lösungsmittel kann jedoch einen zu starken Viskositätsabfall bewirken.

Ein besonders bevorzugtes und in Bezug auf die Stabilisierung der enzymatischen Handgeschirrspülmittel besonders wirksames organisches Lösungsmittel ist das Glycerin sowie das 1,2-Propylenglykol.

Im Hinblick auf die manuelle Anwendung der erfindungsgemäßen Mittel wird in einer bevorzugten Ausführungsform aber auf den Einsatz von organischen Lösungsmitteln verzichtet.

Als Lösungsvermittler insbesondere für Parfüm und Farbstoffe können außer den zuvor beschriebenen Lösungsmitteln beispielsweise auch Alkanolamine sowie Alkylbenzolsulfonate mit 1 bis 3 Kohlenstoffatomen im Alkylrest eingesetzt werden.

Neben den bisher genannten Komponenten können die erfindungsgemäßen Mittel weitere Inhaltsstoffe enthalten. Hierzu zählen beispielsweise Salze, Additive zur Verbesserung des Ablauf- und Trocknungsverhaltens, zur Einstellung der Viskosität, zur Stabilisierung sowie weitere in Handgeschirrspülmitteln übliche Hilfs- und Zusatzstoffe, etwa UV-Stabilisatoren, Parfüm, Perlglanzmittel (INCI Opacifying Agents; beispielsweise Glykoldistearat, z.B. Cutina® AGS der Fa. Cognis, bzw. dieses enthaltende Mischungen, z.B. die Euperlane® der Fa. Cognis), Farbstoffe, Korrosionsinhibitoren, Konservierungsmittel (z.B. das technische auch als Bronopol bezeichnete 2-Brom-2-nitropropan-1,3-diol (CAS 52-51-7), das beispielsweise als Myacide® BT oder als Boots Bronopol BT von der Firma Boots gewerblich erhältlich ist), organische Salze, Desinfektionsmittel, Enzyme, pH-Stellmittel sowie Hautgefühl-verbessernde oder pflegende Additive (z.B. dermatologisch wirksame Substanzen wie Vitamin A, Vitamin B2, Vitamin B12, Vitamin C, Vitamin E, D-Panthenol, Sericerin, Collagen-Partial-Hydrolysat, verschiedene pflanzliche Protein-Partial-Hydrolysate, Proteinhydrolysat-Fettsäure-Kondensate, Liposome, Cholesterin, pflanzliche und tierische Öle wie z.B. Lecithin, Sojaöl, usw., Pflanzenextrakte wie z.B. Aloe Vera, Azulen, Hamamelisextrakte, Algenextrakte, usw., Allantoin, A.H.A.-Komplexe), die in Mengen von üblicherweise nicht mehr als 5 Gew.-% enthalten sein können.

Erfindungsgemäße Reinigungsmittel können Salze enthalten, wobei insbesondere wasserlösliche Salze bevorzugt sind. Hierzu zählen vor allem anorganische Salze, die vorzugsweise ausgewählt sind aus der Gruppe umfassend farblose wasserlösliche Halogenide, Sulfate, Sulfite, Carbonate, Hydrogencarbonate, Nitrate, Nitrite, Phosphate und/oder Oxide der Alkalimetalle, der Erdalkalimetalle, des Aluminiums und/oder der Übergangsmetalle sowie Gemische derselben, besonders bevorzugt aus der Gruppe der Halogenide und Sulfate der Alkalimetalle sowie Gemische derselben, insbesondere aus der Gruppe Natriumchlorid, Kaliumchlorid, Natriumsulfat, Kaliumsulfat sowie Gemische derselben. Besonders bevorzugt ist Natriumchlorid.

Ebenso sind wasserlösliche organische Salze erfindungsgemäß einsetzbar, zum Beispiel Formiat, Acetat, Propionat, Citrat, Malat, Tartrat, Succinat, Malonat, Oxalat, Lactat sowie Gemische derselben. Unter den Gegenionen sind wiederum Alkali-, Erdalkali- oder Übergangsmetallionen, Aluminiumionen sowie Gemische derselben bevorzugt.
Weiterhin können auch Ammoniumsalze allein oder gemeinsam mit anderen Salzen zum Einsatz kommen.

Neben der erfindungsgemäß eingesetzten Amylase können weitere Enzyme eingesetzt werden. Hierzu zählen etwa weitere Amylasen und insbesondere auch Proteasen, aber auch weitere wasch- oder reinigungsaktive Enzyme. Geeignet sind dabei insbesondere solche aus der Klasse der Hydrolasen wie der (Poly)Esterasen, Lipasen, Glykosylhydrolasen, Hemicellulasen, zu denen insbesondere Mannanasen, Xanthanlyasen, Pektinlyasen (=Pektinasen), Pektinesterasen, Pektatlyasen, Xyloglucanasen (=Xylanasen), Pullulanasen und β-Glucanasen gezählt werden, Cutinasen, β-Glucanasen, Oxidasen, Peroxidasen, Mannanasen, Perhydrolasen, Oxireduktasen und/oder Laccasen. Für weitere mögliche Enzyme und Enzym-Zubereitungen wird auf den einschlägigen Stand der Technik zu Wasch- oder Reinigungsmitteln verwiesen.

Zur weiteren Verbesserung des Ablauf- und/oder Trocknungsverhaltens kann das erfindungsgemäße Mittel ein oder mehrere Additive aus der Gruppe der Tenside, der Polymere und der Buildersubstanzen (Builder) enthalten, üblicherweise in einer Menge von 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%, wobei wie oben beschrieben auf Calcium-fällende Buildersubstanzen weitestgehend verzichtet wird.

Als Additive geeignete Polymere sind insbesondere Maleinsäure-Acrylsäure-Copolymer-Na-Salz (Sokalan® CP 5), modifiziertes Polyacrylsäure-Na-Salz (Sokalan® CP 10), modifiziertes Polycarboxylat-Na-Salz (Sokalan® HP 25), Polyalkylenoxid, modifiziertes Heptamethyltrisiloxan (Silwet® L-77), Polyalkylenoxid, modifiziertes Heptamethyltrisiloxan (Silwet® L-7608) sowie Polyethersiloxane (Copolymere von Polymethylsiloxanen mit Ethylenoxid-/Propylenoxidsegmenten (Polyetherblöcken)), vorzugsweise wasserlösliche lineare Polyethersiloxane mit terminalen Polyetherblöcken wie Tegopren® 5840, Tegopren® 5843, Tegopren® 5847, Tegopren® 5851, Tegopren® 5863 oder Tegopren® 5878.

Als Additive geeignete Buildersubstanzen sind insbesondere Polyasparaginsäure-Na-Salz, Ethylendiamintriacetatkokosalkylacetamid (Rewopol® CHT 12), Methylglycindiessigsäure-Tri-Na-Salz (Trilon® ES 9964) und Acetophosphonsäure (Turpinal® SL). Mischungen mit tensidischen oder polymeren Additiven zeigen im Falle von Monawet® MO-84 R2W, Tegopren® 5843 und Tegopren® 5863 Synergismen. Der Einsatz der Tegopren-Typen 5843 und 5863 ist jedoch bei der Anwendung auf harte Oberflächen aus Glas, insbesondere Glasgeschirr, weniger bevorzugt, da diese Silikontenside auf Glas aufziehen können. In einer besonderen Ausführungsform der Erfindung wird auf die genannten Additive verzichtet.

Die für das erfindungsgemäße flüssige Mittel bevorzugte Viskosität liegt bei 20 °C und einer Scherrate von 30 min⁻¹ - gemessen mit einem Viskosimeter vom Typ Brookfield LV DV II und Spindel 25 - im Bereich von 10 bis 10.000 mPa·s, vorzugsweise 600 bis 6.000 mPa·s, insbesondere 1000 bis 5.000 mPa·s. Die Viskosität des erfindungsgemäßen Mittels kann durch Verdickungsmittel erhöht und/oder durch Lösungsmittel verringert werden.

Polymere Verdickungsmittel im Sinne der vorliegenden Erfindung sind die als Polyelektrolyte verdickend wirkenden Polycarboxylate, vorzugsweise Homo- und Copolymerisate der Acrylsäure, insbesondere Acrylsäure-Copolymere wie Acrylsäure-Methacrylsäure-Copolymere, und die Polysaccharide, insbesondere Heteropolysaccharide, sowie andere übliche verdickende Polymere.

Geeignete Polysaccharide bzw. Heteropolysaccharide sind die Polysaccharidgummen, beispielsweise Gummi arabicum, Agar, Alginate, Carrageene und ihre Salze, Guar, Guaran, Tragacant, Gellan, Ramsan, Dextran oder Xanthan und ihre Derivate, z.B. propoxyliertes Guar, sowie ihre Mischungen. Andere Polysaccharidverdicker, wie Stärken oder Cellulosederivate, können alternativ, vorzugsweise aber zusätzlich zu einem Polysaccharidgummi eingesetzt werden, beispielsweise Stärken verschiedensten Ursprungs und Stärkederivate, z.B. Hydroxyethylstärke, Stärkephosphatester oder Stärkeacetate, oder Carboxymethylcellulose bzw. ihr Natriumsalz, Methyl-, Ethyl-, Hydroxyethyl-, Hydroxypropyl-, Hydroxypropyl-methyl- oder Hydroxyethyl-methyl-cellulose oder Celluloseacetat.

Ein bevorzugtes polymeres Verdickungsmittel ist das mikrobielle anionische Heteropolysaccharid Xanthan Gum, das von Xanthomonas campestris und einigen anderen Species unter aeroben Bedingungen mit einem Molekulargewicht von 2-15×10⁶ produziert wird und beispielsweise von der Fa. Kelco unter dem Handelsnamen Keltrol® erhältlich ist, z.B. als cremefarbenes Pulver Keltrol® T (Transparent) oder als weißes Granulat Keltrol® RD (Readily Dispersable).

Als polymere Verdickungsmittel geeignete Acrylsäure-Polymere sind beispielsweise hochmolekulare mit einem Polyalkenylpolyether, insbesondere einem Allylether von Saccharose, Pentaerythrit oder Propylen, vernetzte Homopolymere der Acrylsäure (INCI Carbomer), die auch als Carboxyvinylpolymere bezeichnet werden. Solche Polyacrylsäuren sind u.a. von der Fa. BFGoodrich unter dem Handelsnamen Carbopol® erhältlich, z.B. Carbopol® 940 (Molekulargewicht ca. 4.000.000), Carbopol® 941 (Molekulargewicht ca. 1.250.000) oder Carbopol® 934 (Molekulargewicht ca. 3.000.000).

Besonders geeignete polymere Verdickungsmittel sind aber folgende Acrylsäure-Copolymere: (i) Copolymere von zwei oder mehr Monomeren aus der Gruppe der Acrylsäure, Methacrylsäure und ihrer einfachen, vorzugsweise mit C₁₋₄-Alkanolen gebildeten, Ester (INCI Acrylates Copolymer), zu denen etwa die Copolymere von Methacrylsäure, Butylacrylat und Methylmethacrylat (CAS 25035-69-2) oder von Butylacrylat und Methylmethacrylat (CAS 25852-37-3) gehören und die beispielsweise von der Fa. Rohm & Haas unter den Handelsnamen Aculyn® und Acusol® erhältlich sind, z.B. die anionischen nicht-assoziativen Polymere Aculyn® 33 (vernetzt), Acusol® 810 und Acusol® 830 (CAS 25852-37-3); (ii) vernetzte hochmolekulare Acrylsäurecopolymere, zu denen etwa die mit einem Allylether der Saccharose oder des Pentaerythrits vernetzten Copolymere von C₁₀₋₃₀-Alkylacrylaten mit einem oder mehreren Monomeren aus der Gruppe der Acrylsäure, Methacrylsäure und ihrer einfachen, vorzugsweise mit C₁₋₄-Alkanolen gebildeten, Ester (INCI Acrylates/C10-30 Alkyl Acrylate Crosspolymer) gehören und die beispielsweise von der Fa. BFGoodrich unter dem Handelsnamen Carbopol® erhältlich sind, z.B. das hydrophobierte Carbopol® ETD2623 und Carbopol® 1382 (INCI Acrylates/C10-30 Alkyl Acrylate Crosspolymer) sowie Carbopol® AQUA 30 (früher Carbopol® EX 473).

Der Gehalt an polymerem Verdickungsmittel beträgt üblicherweise nicht mehr als 8 Gew.-%, vorzugsweise zwischen 0,1 und 7 Gew.-%, besonders bevorzugt zwischen 0,5 und 6 Gew.-%. In anderen Ausführungsformen kann der Anteil auch zwischen 1 und 5 Gew.-%, vorzugsweise zwischen 1,5 und 4 Gew.-%, insbesondere bevorzugt zwischen 2 und 2,5 Gew.-% liegen.

In einer besonderen Ausführungsform der Erfindung ist das Mittel jedoch frei von polymeren Verdickungsmitteln.

Zur Stabilisierung des erfindungsgemäßen Mittels, insbesondere bei hohem Tensidgehalt, können ein oder mehrere Dicarbonsäuren und/oder deren Salze zugesetzt werden, insbesondere eine Zusammensetzung aus Na-Salzen der Adipin-, Bernstein- und Glutarsäure, wie sie z.B. unter dem Handelsnamen Sokalan® DSC erhältlich ist. Der Einsatz erfolgt hierbei vorteilhafterweise in Mengen von 0,1 bis 8 Gew.-%, vorzugsweise 0,5 bis 7 Gew.-%, insbesondere 1,3 bis 6 Gew.-% und besonders bevorzugt 2 bis 4 Gew.-%.

Eine Veränderung des Dicarbonsäure(salz)-Gehaltes kann - insbesondere in Mengen oberhalb 2 Gew.-% - zu einer klaren Lösung der Inhaltsstoffe beitragen. Ebenfalls ist innerhalb gewisser Grenzen eine Beeinflussung der Viskosität der Mischung durch dieses Mittel möglich. Weiterhin beeinflusst diese Komponente die Löslichkeit der Mischung. Diese Komponente wird besonders bevorzugt bei hohen Tensidgehalten eingesetzt, insbesondere bei Tensidgehalten oberhalb 30 Gew.-%.

Kann jedoch auf deren Einsatz verzichtet werden, so ist das erfindungsgemäße Mittel vorzugsweise frei von Dicarbonsäure(salze)n.

Als antibakterielle Komponente können erfindungsgemäße Mittel auch elementares Silber und/oder eine Silberverbindung enthalten.

Milchsäure hat auch den Vorteil, dass sie wie Benzoesäure oder auch Salicylsäure als pH-Regulator und/oder Puffer-Substanz die antibakterielle Wirkung des Silbers und/oder der Silberverbindung unterstützen bzw. verstärken kann

Der pH-Wert des erfindungsgemäßen flüssigen Mittels kann aber auch mittels üblicher pH-Regulatoren, beispielsweise Säuren wie Mineralsäuren oder Citronensäure und/oder Alkalien wie Natrium- oder Kaliumhydroxid, eingestellt werden, wobei - insbesondere bei gewünschter Haut- und Handverträglichkeit - ein Bereich von 6,0 bis 9,0, vorzugsweise 7,0 bis 8,0 bevorzugt ist (gemessen unverdünnt und bei 20°C). Zur Einstellung und/oder Stabilisierung des pH-Werts kann das erfindungsgemäße Mittel ein oder mehrere Puffer-Substanzen (INCI Buffering Agents) enthalten, üblicherweise in Mengen von 0,001 bis 5 Gew.-%, vorzugsweise 0,005 bis 3 Gew.-%, insbesondere 0,01 bis 2 Gew.-%, besonders bevorzugt 0,05 bis 1 Gew.-%, äußerst bevorzugt 0,1 bis 0,5 Gew.-%, beispielsweise 0,2 Gew.-%. Bevorzugt sind Puffer-Substanzen, die zugleich Komplexbildner oder sogar Chelatbildner (Chelatoren, INCI Chelating Agents) sind. Besonders bevorzugte Puffer-Substanzen sind die Citronensäure bzw. die Citrate, insbesondere die Natrium- und Kaliumcitrate, beispielsweise Trinatriumcitrat·2 H₂O und Trikaliumcitrat·H₂O.

Ein flüssiges Mittel kann weiterhin Hydrotrope enthalten. Hierbei handelt es sich um Löslichkeitsvermittler. Geeignete Hydrotrope sind beispielsweise Harnstoff, Butylglycol, oder aliphatische kurzkettige anionische oder amphotere Lösungsvermittler.

In einer Ausführungsform wird das erfindungsgemäße flüssige Reinigungsmittel zur Reinigung harter Oberflächen, also insbesondere zur Reinigung von verschmutztem Geschirr, in Form eines Schaums entweder direkt auf die zu reinigende Oberfläche oder auf einen Schwamm, ein Tuch, eine Bürste oder ein anderes, gegebenenfalls angefeuchtetes, Reinigungshilfsmittel aufgetragen. Zur Schaumerzeugung eignet sich in besonderer Weise ein manuell aktivierter Sprühspender, insbesondere ausgewählt aus der Gruppe umfassend Aerosolsprühspender, selbst Druck aufbauende Sprühspender, Pumpsprühspender und Triggersprühspender, insbesondere Pumpschaumspender, wie sie beispielsweise von der Firma Airspray, der Firma Taplast, der Firma Keltec oder auch der Daiwa Can Company angeboten werden. Neben Triggerflaschen eignen sich auch Pumpsprühspender und Triggersprühspender mit einem Behälter aus Polyethylen, Polypropylen oder Polyethylenterephthalat. Solche Triggerflaschen werden beispielsweise von der Firma Afa-Polytec angeboten. Der Sprühkopf ist vorzugsweise mit einer Schaumdüse ausgestattet. Daneben kann das Mittel auch unter Zusatz eines geeigneten Treibmittels (z.B. n-Butan, ein Propan/-Butan-Gemisch, Kohlendioxid, Stickstoff oder ein CO₂/N₂-Gemisch) in eine entsprechende Aerosolsprühflasche gefüllt werden. Ein solcher Sprühspender ist jedoch weniger bevorzugt.

Dementsprechend kann das erfindungsgemäße Mittel in Form eines Erzeugnisses aus dem erfindungsgemäßen Mittel und einem Sprüh- oder Schaumspender, insbesondere Pumpschaumspender, in Verkehr gebracht werden.

Zur manuellen Reinigung einer harten Oberfläche kann das erfindungsgemäße flüssige Reinigungsmittel entweder direkt, das heißt unverdünnt, beispielsweise mittels eines Schwamms, auf die zu reinigende Oberfläche aufgebracht und anschließend mit Wasser wieder entfernt werden.

Alternativ kann das erfindungsgemäße Handgeschirrspülmittel zunächst mit Wasser auf Konzentrationen von 1 : 1 bis 1 : 1000 verdünnt und anschließend die erhaltene Reinigungslösung mit der zu reinigenden Oberfläche in Kontakt gebracht werden.

Im Folgenden soll die vorliegende Erfindung durch Beispiele näher beschrieben werden. Diese Beispiele sind jedoch keinesfalls limitierend für den Gegenstand der vorliegenden Erfindung und dienen lediglich zur Veranschaulichung für den Fachmann.

### Ausführungsbeispiele

Es wurde eine erfindungsgemäße Formulierung E1 sowie eine Vergleichsformulierung V1 hergestellt. E1 enthielt hierbei eine erfindungsgemäße Amylase ("Amylase 1"), während in V1 eine handelsübliche Amylase ("Amylase 2", Stainzyme 12L) eingesetzt wurde. Die Zusammensetzungen sind in der nachfolgenden Tabelle wiedergegeben, wobei alle Mengenangaben in Gew.-% des eingesetzten Rohstoffs sind.

| | E1 | V1 |
|---|---|---|
| Fettalkoholethersulfat | 10 | 10 |
| Cocoamidopropylbetain | 4 | 4 |
| Kaliumacetat | 0,2 | 0,2 |
| Parfüm | 0,2 | 0,2 |
| Konservierungsmittel | 0,1 | 0,1 |
| Amylase 1 | 0,2 | -- |
| Amylase 2 | -- | 0,2 |
| Salze | 1 | 1 |
| Wasser | Ad 100% | Ad 100% |

Die beiden Formulierungen wurden hinsichtich ihrer Wirkung an eingetrockneter Stärke bei verschiedenen Temperaturen überprüft:
Edelstahlbleche (10 x 15 cm), auf denen sich jeweils 1,2 g einer eingetrockneten Stärkemischung (je 5 g Reis-, Mais-, Weizen- und Kartoffelstärke gequollen in 500 ml Wasser) wurden für 20 Minuten in einer wässrigen Lösung aus einer der beiden Reinigungsmittelformulierungen (5 g/l) eingeweicht, wobei jeweils Wasser verschiedener Temperaturen (40°C/30°C/20°C) eingesetzt wurde. Anschließend wurden die Edelstahlplatten unter fließendem Wasser ohne Wischen abgespült und getrocknet. Anschließend wurde die Stärkeentfernung gravimetrisch bestimmt. Das Ergebnis ist in der nachfolgenden Tabelle wiedergegeben:

| | E1 | V1 |
|---|---|---|
| 40°C | 97% | 93% |
| 30°C | 66% | 39% |
| 20°C | 65% | 8% |

Die erfindungsgemäße Formulierung weist somit gegenüber dem Stand der Technik klare Leistungsvorteile insbesondere bei Spültemperaturen unterhalb von 40°C auf.

## Patentansprüche

1. Flüssiges Reinigungsmittel für harte Oberflächen, insbesondere zum manuellen Geschirrspülen enthaltend eine α-Amylase, **dadurch gekennzeichnet, dass** die α-Amylase zu der in SEQ ID NO.1 angegeben Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 Deletionen aufweist, wobei
die α-Amylase Deletionen an mindestens zwei Positionen ausgewählt aus den Positionen 180+181, 181+182, 182+183 und 183+184 in der Zählung gemäß SEQ ID NO. 1 aufweist, und wobei das flüssige Reinigungsmittel Kaliumionen enthält und der Anteil der Kaliumionen an der Gesamtzusammensetzung 0,025 bis 0,25 Gew.-% beträgt.

2. Flüssiges Reinigungsmittel gemäß Anspruch 1, wobei die α-Amylase die Deletionen H183* + G184* in der Zählung gemäß SEQ ID NO. 1 aufweist.

3. Flüssiges Reinigungsmittel gemäß Anspruch 1 bis 2, wobei die α-Amylase in der Zählung gemäß SEQ ID NO. 1 weiterhin Aminosäuresubstitutionen an einer oder mehreren der Positionen 405, 421, 422 und 428 aufweist.

4. Flüssiges Reinigungsmittel gemäß Anspruch 1 bis 3, wobei die α-Amylase in der Zählung gemäß SEQ ID NO. 1 die Substitutionen I405L; A421H, A422P und A428T aufweist.

5. Flüssiges Reinigungsmittel gemäß einem der vorstehenden Ansprüche, **dadurch gekennzeichnet, dass** es einen Gehalt an α-Amylase von 0,05 bis 1,0 Gew.-% aufweist, vorzugsweise von 0,1 bis 0,4 Gew.-%.

6. Flüssiges Reinigungsmittel gemäß einem der vorstehenden Ansprüche, **dadurch gekennzeichnet, dass** es ein oder mehrere Tenside enthält.

7. Flüssiges Reinigungsmittel gemäß Anspruch 6, **dadurch gekennzeichnet, dass** es eine Tensidkombination aus mindestens einem anionischen Tensid, vorzugsweise mindestens einem Alkylethersulfat, und mindestens einem amphoteren Tensid, vorzugsweise mindestens einem Betain-Tensid, enthält.

8. Flüssiges Reinigungsmittel gemäß einem der Ansprüche 6 oder 7, **gekennzeichnet durch** einen Tensidgehalt von 5 bis 50 Gew.-%, vorzugsweise 10 bis 40 Gew.-%.

9. Flüssiges Reinigungsmittel gemäß einem der Ansprüche 1 bis 8, **dadurch gekennzeichnet, dass** der Anteil der Kaliumionen an der Gesamtzusammensetzung 0,05 bis 0,125 Gew.-% beträgt.

10. Flüssiges Reinigungsmittel gemäß einem der vorstehenden Ansprüche, **dadurch gekennzeichnet, dass** es einen pH-Wert von 6,0 bis 9,0, bevorzugt 7,0 bis 8,0 aufweist (unverdünnt, 20°C).

11. Flüssiges Reinigungsmittel gemäß mindestens einem der vorhergehenden Ansprüche, **dadurch gekennzeichnet, dass** es weitere übliche Inhaltsstoffe von Wasch- oder Reinigungsmitteln enthält, vorzugsweise ausgewählt aus der Gruppe umfassend Salze, Builder, Lösungsmittel, weitere Tenside, Additive zur Verbesserung des Ablauf- und Trocknungsverhaltens, Verdickungsmittel, Additive zur Stabilisierung, Parfüm, Perlglanzmittel, Farbstoffe, UV-Stabilisatoren, Korrosionsinhibitoren, Konservierungsmittel, Desinfektionsmittel, Enzyme, pH-Stellmittel, Bitterstoffe, antimikrobielle Wirkstoffe, Hautgefühl-verbessernde oder pflegende Additive sowie Gemische derselben.

12. Verwendung eines flüssigen Reinigungsmittels gemäß mindestens einem der vorhergehenden Ansprüche zum manuellen Spülen von Geschirr.

13. Verfahren zur Reinigung von harten Oberflächen, insbesondere zur manuellen Reinigung von Geschirr, umfassend die Verwendung eines flüssigen Reinigungsmittels gemäß mindestens einem der Ansprüche 1 bis 11.

## Claims

1. A liquid cleaning agent for hard surfaces, in particular for manual dishwashing, containing an α-amylase, **characterized in that** the α-amylase is at least 89% and, in order of increasing preference, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, 99.5% and up to 100% identical to the sequence shown in SEQ ID NO. 1 over the entire length thereof and has deletions at one or more of the positions 180, 181, 182, 183 and 184 in the numbering according to SEQ ID NO. 1, the α-amylase having deletions at at least two positions selected from the positions 180+181, 181+182, 182+183 and 183+184 in the numbering according to SEQ ID NO. 1, and the liquid cleaning agent containing potassium ions and the proportion of potassium ions in the total composition being 0.025 to 0.25 wt.%.

2. The liquid cleaning agent according to claim 1, wherein the α-amylase has the deletions H183* + G184* in the numbering according to SEQ ID NO. 1.

3. The liquid cleaning agent according to claim 1 to 2, wherein the α-amylase additionally has amino acid substitutions at one or more of the positions 405, 421, 422 and 428 in the numbering according to SEQ ID NO. 1.

4. The liquid cleaning agent according to claim 1 to 3, wherein the α-amylase has the substitutions I405L; A421H, A422P and A428T in the numbering according to SEQ ID NO. 1.

5. The liquid cleaning agent according to one of the preceding claims, **characterized in that** it has an α-amylase content of 0.05 to 1.0 wt.%, preferably 0.1 to 0.4 wt.%.

6. The liquid cleaning agent according to one of the preceding claims, **characterized in that** it contains one or more surfactants.

7. The liquid cleaning agent according to claim 6, **characterized in that** it contains a surfactant combination of at least one anionic surfactant, preferably at least one alkyl ether sulfate, and at least one amphoteric surfactant, preferably at least one betaine surfactant.

8. The liquid cleaning agent according to one of claims 6 or 7, **characterized by** a surfactant content of 5 to 50 wt.%, preferably 10 to 40 wt.%.

9. The liquid cleaning agent according to one of claims 1 to 8, **characterized in that** the proportion of potassium ions in the total composition is 0.05 to 0.125 wt.%.

10. The liquid cleaning agent according to one of the preceding claims, **characterized in that** it has a pH of 6.0 to 9.0, preferably 7.0 to 8.0 (undiluted, 20°C).

11. The liquid cleaning agent according to at least one of the preceding claims, **characterized in that** it contains additional customary ingredients of washing or cleaning agents, preferably selected from the group comprising salts, builders, solvents, additional surfactants, additives for improving the flow and drying behavior, thickening agents, stabilizing additives, perfume, pearlescing agents, dyes, UV stabilizers, corrosion inhibitors, preservatives, disinfectants, enzymes, pH adjusters, bitterns, antimicrobial active ingredients, additives for improving skin feel or nourishing additives and mixtures thereof.

12. The use of a liquid cleaning agent according to at least one of the preceding claims for manually rinsing dishes.

13. A method for cleaning hard surfaces, in particular for manually cleaning dishes, comprising the use of a liquid cleaning agent according to at least one of claims 1 to 11.

## Revendications

1. Agent nettoyant liquide pour surfaces dures, en particulier pour le lavage manuel de vaisselle, contenant une α-amylase, **caractérisé en ce que** l'α-amylase, par rapport à la séquence donnée dans SEQ ID NO.1, est identique sur toute sa longueur à raison d'au moins 89 % et de manière plus préférée d'au moins 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 %, 99 %, 99,5% et jusqu'à 100 %, et présente des délétions au niveau de l'une ou plusieurs des positions 180, 181, 182, 183 et 184 dans le décompte selon SEQ ID NO. 1, dans lequel
les α-amylases présentent des délétions au niveau d'au moins deux positions choisies parmi les positions 180+181, 181+182, 182+183 et 183+184 dans le décompte selon SEQ ID NO. 1, et
l'agent nettoyant liquide contenant des ions potassium et la teneur en ions potassium par rapport à la composition totale étant de 0,025 à 0,25 % en poids.

2. Agent nettoyant liquide selon la revendication 1, dans lequel l'α-amylase présente les délétions H183 * + G184 * dans le décompte selon SEQ ID NO. 1.

3. Agent nettoyant liquide selon les revendications 1 à 2, dans lequel l'α-amylase présente en outre, dans le décompte selon SEQ ID NO. 1, des substitutions d'acides aminés au niveau d'une ou de plusieurs des positions 405, 421, 422 et 428.

4. Agent nettoyant liquide selon les revendications 1 à 3, dans lequel l'α-amylase présente, dans le décompte selon SEQ ID NO. 1, les substitutions 1405L ; A421H, A422P et A428T.

5. Agent nettoyant liquide selon l'une quelconque des revendications précédentes, **caractérisé en ce qu'**il présente une teneur en α-amylase comprise entre 0,05 et 1,0 % en poids, de préférence entre 0,1 et 0,4 % en poids.

6. Agent nettoyant liquide selon l'une des revendications précédentes, **caractérisé en ce qu'**il contient un ou plusieurs tensioactifs.

7. Agent nettoyant liquide selon la revendication 6, **caractérisé en ce qu'**il contient une combinaison tensioactive d'au moins un tensioactif anionique, de préférence au moins un alkyl éther-sulfate, et au moins un tensioactif amphotère, de préférence au moins un tensioactif bétaïne.

8. Agent nettoyant liquide selon l'une des revendications 6 ou 7, **caractérisé par** une teneur en tensioactif de 5 à 50 % en poids, de préférence de 1 à 40 % en poids.

9. Agent nettoyant liquide selon l'une des revendications 1 à 8, **caractérisé en ce que** la proportion d'ions potassium par rapport à la composition totale est de 0,05 à 0,125 % en poids.

10. Agent nettoyant liquide selon l'une des revendications précédentes, **caractérisé en ce qu'**il présente un pH de 6,0 à 9,0, de préférence de 7,0 à 8,0 (non dilué, 20°C).

11. Agent nettoyant liquide selon au moins l'une des revendications précédentes, **caractérisé en ce qu'**il contient d'autres ingrédients usuels d'agents de lavage ou de nettoyage, choisis de préférence dans le groupe comprenant les sels, les adjuvants, les solvants, les autres tensioactifs, les additifs pour l'amélioration du drainage et du séchage, les agents épaississants, les additifs pour la stabilisation, le parfum, les agents nacrés, les colorants, les stabilisants UV, les inhibiteurs de corrosion, les agents de conservation, les agents désinfectants, les enzymes, les ajusteurs de pH, les agents amérisants, les agents antimicrobiens, les additifs soignant ou améliorant la sensation cutanée, ainsi que les mélanges de ceux-ci.

12. Utilisation d'un agent nettoyant liquide selon l'une au moins des revendications précédentes pour le lavage manuel de la vaisselle.

13. Procédé pour le nettoyage de surfaces dures, en particulier pour le nettoyage manuel de vaisselle, comprenant l'utilisation d'un agent nettoyant liquide selon au moins l'une des revendications 1 à 11.
